# EUROPEAN PATENT SPECIFICATION

(11) **EP 4 223 354 B1**
(45) Date of publication and mention of the grant of the patent: **21.02.2024**
(21) Application number: 22167155.5
(22) Date of filing: 07.04.2022
(51) Int. Cl.: A61M 37/00

(54) **STAMP TYPE FLUID DELIVERY CONTAINER WITH MICRONEEDLE**
STEMPELARTIGER FLUIDAUSGABEBEHÄLTER MIT MIKRONADEL
RÉCIPIENT DE DISTRIBUTION DE FLUIDE DE TYPE TAMPON À MICRO-AIGUILLE

(30) Priority: 07.02.2022 KR 20220015262
(43) Date of publication of application: 09.08.2023
(73) Proprietor: Han, Sangbum, Uichang-gu Changwon-si Gyeongsangnam-do (KR)
(72) Inventor: Han, Sangbum, Uichang-gu Changwon-si Gyeongsangnam-do (KR)
(74) Representative: Cabinet Chaillot

(56) References cited:
- EP-A1- 3 287 037
- CN-A- 109 078 259
- KR-A- 20210 051 125
- KR-U- 20120 007 011

## Description

### CROSS REFERENCE TO RELATED APPLICATION

The present application claims priority to Korean Patent Application No. 10-2022-0015262, filed February 07, 2022.

### BACKGROUND OF THE INVENTION

### Field of the Invention

The present disclosure relates to a stamp type fluid delivery container with microneedles, the container being used to inject fluid for skin or scalp care, treatment, etc. while it is tapped on the skin or scalp. In more particularly, the present disclosure relates to a stamp type fluid delivery container with microneedles, the container having a simple structure without a specific nozzle opening/closing structure and being used to uniformly and smoothly supply fluid to the microneedles. Some documents, like EP-A-3287037 disclose examples of containers with microneedles.

### Description of the Related Art

In general, fluid that is used for skin or scalp care, treatment, etc. is directly supplied to the skin or the scalp, but there is a problem that it is difficult to expect a great effect because the fluid spreads on the skin or the outer layer of the scalp.

In order to solve this problem, several types of fluid delivery containers that make fluid permeate even into a dermis (inner skin) other than the outer layer of a skin or a scalp using microneedles have been proposed.

For example, according to Korean Patent No. 10-1993706, a connecting housing is coupled to the upper portion of a containing member (container) keeping fluid, a needle housing having microneedles inserted in slots formed in parallel is coupled to the connecting housing, and a cover bar and a nozzle bar are inserted in a nozzle hole at the center of the connecting housing to be able to be elastically moved up and down, so when the nozzle bar is pressed on the skin or the scalp, the nozzle hole is opened, and fluid is supplied through a medical fluid, the connecting housing, or the microneedles in the slots of the needle housing to permeate into the dermis (inner skin) of the skin or the scalp.

However, according to the fluid delivery container, the nozzle opening/closing structure and the connecting housing are separately formed, so the structure is complicated, and the fluid supplied through the nozzle hole of the connecting housing is supplied to the entire needle housing through the front space of the connecting housing rather than being directly supplied to the slots of the needle housing, so there is a problem that fluid is not uniformly and smoothly supplied to the slots of the needle housing.

Further, according to Korean Patent No. 10-1776154, a base is coupled to an upper portion of a containing member (container) without a nozzle structure, a cover microneedles inserted in slots formed in parallel is coupled to the base, and a sponge member is coupled to an upper portion of the cover such that fluid permeates into the dermis (inner skin) of the skin or the scalp through the microneedles without a nozzle opening/closing structure.

However, according to this fluid delivery container, there is no nozzle opening/closing structure, but a specific member such as the base is further coupled, so the structure is complicated. Further, the fluid in the containing member is directly supplied to a fluid channel hole formed separately from the slots of the cover, so there is a problem that fluid is smoothly supplied, but the fluid is not supplied by an appropriate amount and is excessively supplied.

Further, there is another problem that since the fluid channel hole of the cover keeps open even when the fluid delivery container is kept or carried, fluid flows and leaks outside.

### Documents of Related Art

(Patent Document 1) Korean Patent No. 10-1776154 (2017. 09. 01)
(Patent Document 2) Korean Patent No. 10-1993706 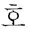 (2019. 06. 21)

### SUMMARY OF THE INVENTION

The present disclosure has been made in an effort to solve the generally problems in the related art described above and an objective of the present disclosure is to provide a fluid delivery container that is used to make fluid for skin or scalp care, treatment, etc. permeate into the skin or the scalp through microneedles, has a simple structure without a nozzle opening/closing structure, uniformly and smoothly supplies fluid to the microneedles, and prevents the possibility of leakage of fluid at normal times.

A stamp type fluid delivery container with microneedles of the present disclosure includes: a container body configured to keep fluid therein, having a coupling portion on an outer side of an upper portion thereof, and having a coupling groove inside the upper portion; a needle housing having a body that is inserted in the coupling groove of the container body and has slots that are vertically formed through the body and arranged to the left and right in parallel and in which microneedles are inserted, respectively; a sealing cover installed on a top of the container body and preventing leakage of fluid coming out of the slots of the needle housing; and a closing cap coupled to the coupling portion of the container body, in which a fluid discharge hole is formed in an oblong shape on a bottom of the coupling groove of the container body across the slots of the needle housing, and an air hole preventing leakage of fluid in the container body and enabling air to flow is formed in an upper portion of the coupling portion.

According to the present disclosure, a needle seat groove may be formed at a center of a bottom of the sealing cover such that upper ends of the microneedles are positioned therein.

According to the present disclosure, a bottom cap made of rubber may be coupled to a bottom of the container body to be able to be opened and closed for back-filling of fluid.

According to the present disclosure, a needle housing having microneedles coupled in slots formed to the left and right in parallel is coupled to a coupling groove at the upper portion of a container body, a fluid discharge hole is formed in an oblong shape on the bottom of the coupling groove across the slots of the needle housing, and an air hole through which air can flow without leakage of fluid is formed in the upper portion of the container body. Accordingly, it is possible to uniformly and smoothly supply fluid not excessively, but appropriately to the microneedles even without a nozzle opening/closing structure and it is also possible to use the fluid delivery container without the possibility of leakage of fluid at normal times. Therefore, there is an effect that it is possible to increase productivity in manufacturing, reliability in use, and economics.

### BRIEF DESCRIPTION OF THE DRAWINGS

The above and other objectives, features and other advantages of the present invention will be more clearly understood from the following detailed description when taken in conjunction with the accompanying drawings, in which:
FIG. 1 is a perspective view showing the external appearance of a container of the present disclosure;
FIG. 2 is a half cross-sectional perspective view of FIG. 1.
FIG. 3 is a front cross-sectional configuration view of FIG. 1;
FIG. 4 is an enlarged view of the part A of FIG. 3;
FIG. 5 is an exploded perspective view of main parts of FIG. 2;
FIG. 6 is a plan view showing a structure in which a needle housing is coupled to a container body of the present disclosure; and

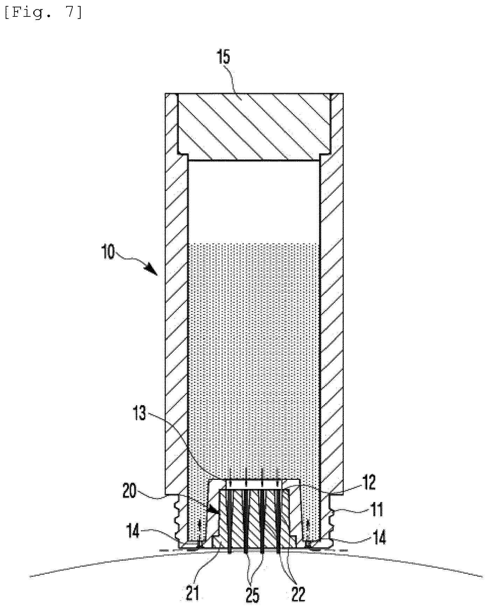

FIG. 7 is a cross-sectional configuration view showing a use state of the container of the present disclosure.

### DETAILED DESCRIPTION OF THE INVENTION

Hereinafter, exemplary embodiments of the present disclosure will be described in detail with reference to the accompanying drawings.

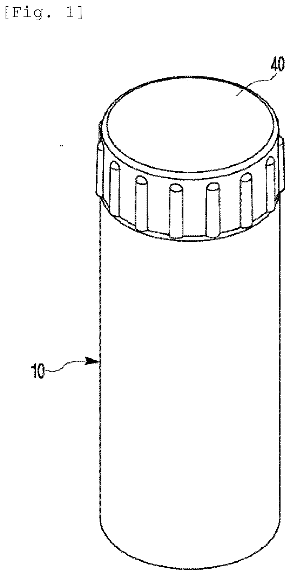

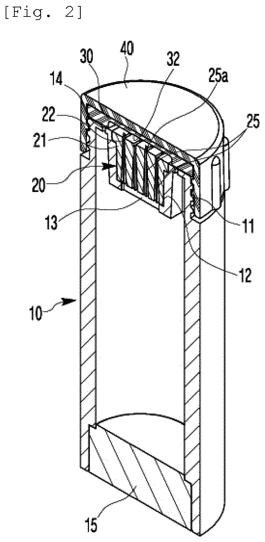

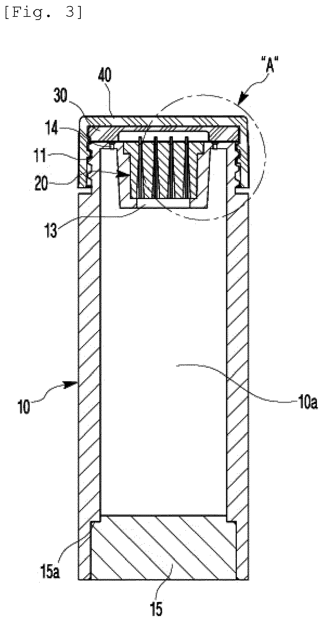

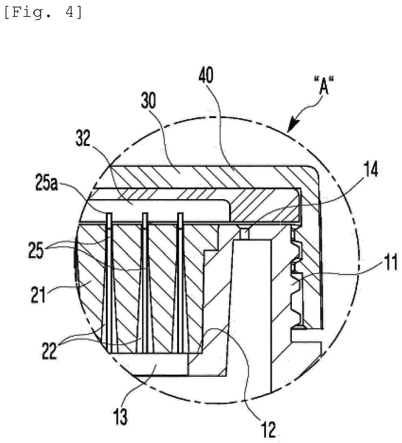

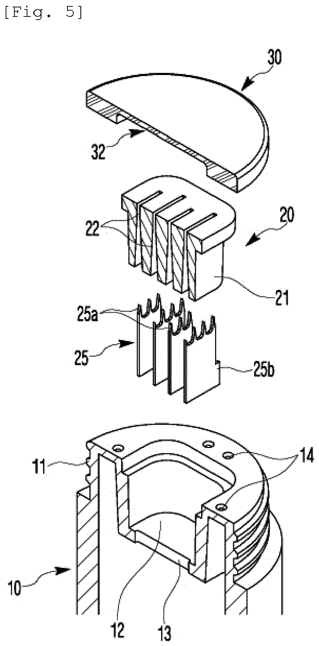

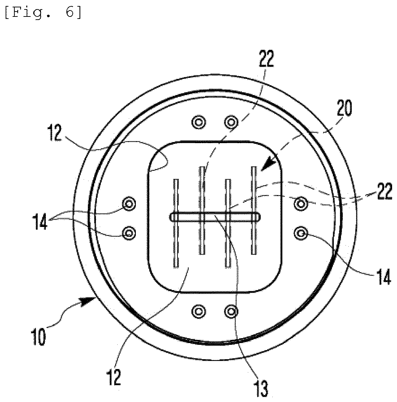

A stamp type fluid delivery container with microneedles of the present disclosure, as shown in FIGS. 1 to 6, includes a container body 10, a needle housing 20, a sealing cover 30, and a closing cap 40.

The container body 10 keeps fluid in the internal space thereof and has a threaded coupling portion 11 on the outer side of the upper portion and a coupling groove 12 inside the upper portion.

The coupling groove 12 may be stepped up and down and may be formed in a rectangular shape when seen from above.

The needle housing 20 has a rectangular body 21 corresponding to the coupling groove 12 of the container body and is fixed and coupled by being forcibly inserted in the coupling groove. Further, the needle housing 20 has an up-down stepped structure, which corresponds to the up-down step of the coupling groove, on the outer side.

Slots 22 are vertically formed and arranged to the left and right in parallel in the needle housing 20 and plate-shaped microneedles 25 are inserted in the slots, respectively.

Sub-microneedles 25a protruding from the upper end of each of the microneedles 25 protrude upward out of the needle housing, the slots 22 of the needle housing are tapered such that the width increases downward, and holding steps 25b formed on both sides of the lower portion of the microneedles are retained in the slots 22. Accordingly, the microneedles are not separated upward out of the slots 22 and fluid is smoothly supplied to the slots 22.

The sealing cover 30 is made of rubber and is installed to cover the entire upper portion of the coupling portion 11 of the container body, thereby preventing fluid flowing out through the slots 22 of the needle housing from leaking to the outside. A needle seat groove 32 in which the sub-microneedles 25a at the upper ends of the microneedles 25 are inserted is formed at the center portion of the bottom of the sealing cover.

The closing cap 40 is thread-fastened to the coupling portion 11 of the container body and presses the sealing cover 30 so that the sealing cover is operated for sealing.

In particular, a fluid discharge hole 13 is formed in an oblong shape on the bottom of the coupling groove 12 of the container body to cross the slots 22 of the needle housing and an air hole 14 is formed in the upper portion of the coupling portion 11 so that air can flow while the fluid in the container body is prevented from leaking. Accordingly, fluid is smoothly supplied to the microneedles 25 by a simple structure without a separate nozzle opening/closing structure.

The fluid discharge hole 13 is not formed through the bottom of the coupling groove 12 of the container body and is formed in an oblong shape, thereby preventing excessive supply of fluid. Further, the fluid discharge hole 13 is formed across the slots 22 of the needle housing so that fluid is uniformly supplied to the slots of the needle housing.

A bottom cap 15 made of rubber is coupled to the bottom of the container body 10 by forcible-fitting to be able to be open and closed for back-filling of fluid.

Though not stated about, reference numeral '10a' indicates the internal space of the container body and reference numeral '15a' indicates a stepped groove formed at a lower portion of the container body to couple the bottom cap.

The operation and function of the present disclosure having the configuration described above are described hereafter.

First, a process of assembling the fluid delivery container of the present disclosure is described. The plate-shaped microneedles 25 are inserted into the slots 22 formed to the left and right in parallel in the needle housing 20 from the bottoms of the slots 22 and the needle housing 20 is inserted into the coupling groove 12 formed inside the upper portion of the container body 10.

Since the oblong fluid discharge hole 13 is formed on the bottom of the coupling groove 12 of the container body, the slots 22 of the needle housing 20 are positioned across the fluid discharge hole 13.

In this state, the sealing cover 30 is installed on the top of the container body without interfering with the sub-microneedles 25a at the upper ends of the microneedles because the needle seat groove 32 is formed at the center portion of the bottom of the sealing cover.

Then, the closing cap 40 is coupled to the coupling portion 11 on the outer side of the upper portion of the container body.

In this state, the bottom cap 15 coupled to the bottom of the container body is separated, the container body 10 is filled with fluid from the back, and then the bottom cap 15 is coupled back for closing.

Since the sealing cover 30 is coupled to the upper portion of the container body 10 assembled in this way in the present disclosure, fluid in the container body 10 is prevented from leaking to the outside.

In order to use the fluid delivery container of the present disclosure, a user separates the closing cap 40 from the upper portion of the container body 10 and separates also the sealing cover 30 at the upper portion of the container body, and then the user can use the fluid delivery container.

In this prepared state, the user taps the sub-microneedles 25a of the microneedles coupled to the upper portion of the container body 10 on a scalp, a skin, etc., whereby the fluid in the container body is supplied to the microneedles and injected into micro holes formed by the sub-microneedles 25a.

Describing this process in more detail, when the fluid in the container body 10 is supplied through the fluid discharge hole 13 formed in an oblong shape on the bottom of the coupling groove 12, the fluid is supplied also to the slots 22 of the needle housing which are formed in parallel across the fluid discharge hole 13.

In particular, since the fluid discharge hole 13 is not formed through the bottom of the coupling groove 12 of the container body and is formed in an oblong shape, excessive supply of fluid is prevented. Further, since the fluid discharge hole 13 is formed across the slots 22 of the needle housing, fluid is uniformly supplied to the slots of the needle housing.

Further, since the air hole 14 is formed in the upper portion of the container body 10, external air flows into the container body and enables the fluid in the container body to be smoothly discharged through the fluid discharge hole 13 without leakage of the fluid.

As described above, an appropriate amount of fluid is uniformly supplied to the slots 22 of the needle housing through the fluid discharge hole 13 of the container body and is injected through the microneedles 25 coupled in the slots 22.

Therefore, according to the present disclosure, since the needle housing 20 having the microneedles 25 is coupled to the coupling groove 12 at the upper portion of the container body 10, the fluid discharge hole 13 is formed in an oblong shape on the bottom of the coupling groove 12 across the slots 22 of the needle housing, and the air hole 14 through which air can flow without leakage of fluid is formed in the upper portion of the container body, it is possible to uniformly and smoothly supply fluid not excessively, but appropriately to the microneedles even without a nozzle opening/closing structure and it is also possible to use the fluid delivery container without the possibility of leakage of fluid at normal times.

## Claims

1. A stamp type fluid delivery container with microneedles, the stamp type fluid delivery container comprising:
a container body (10) configured to keep fluid therein, having a coupling portion (11) on an outer side of an upper portion thereof, and having a coupling groove (32) inside the upper portion;
a needle housing (20) having a body that is inserted in the coupling groove of the container body and has slots that are vertically formed through the body and arranged to the left and right in parallel and in which microneedles are inserted, respectively;
a sealing cover (30) installed on a top of the container body and preventing leakage of fluid coming out of the slots of the needle housing; and
a closing cap (40) coupled to the coupling portion of the container body,
wherein a fluid discharge hole (13) is formed in an oblong shape on a bottom of the coupling groove of the container body across the slots of the needle housing, and an air hole preventing leakage of fluid in the container body and enabling air to flow is formed in an upper portion of the coupling portion.

2. The stamp type fluid delivery container of claim 1, wherein a needle seat groove is formed at a center of a bottom of the sealing cover such that upper ends of the microneedles are positioned therein.

3. The stamp type fluid delivery container of claim 1, wherein a bottom cap made of rubber is coupled to a bottom of the container body to be able to be opened and closed for back-filling of fluid.

## Patentansprüche

1. - Stempelartiger Fluidzufuhrbehälter mit Mikronadeln, der stempelartige Fluidzufuhrbehälter umfassend:
einen Behälterkörper (10), der konfiguriert ist, um ein Fluid darin zu halten, der einen Kupplungsabschnitt (11) an einer Außenseite eines oberen Abschnitts davon aufweist und eine Kupplungsnut (32) innerhalb des oberen Abschnitts aufweist;
ein Nadelgehäuse (20), das einen Körper aufweist, der in die Kupplungsnut des Behälterkörpers eingesetzt wird und Schlitze aufweist, die vertikal durch den Körper gebildet und links und rechts parallel angeordnet sind und in die jeweils Mikronadeln eingesetzt werden;
eine Dichtungsabdeckung (30), die auf einer Oberseite des Behälterkörpers installiert ist und ein Austreten von Fluid aus den Schlitzen des Nadelgehäuses verhindert; und
eine Verschlusskappe (40), die an den Kupplungsabschnitt des Behälterkörpers gekoppelt ist,
wobei ein Fluidauslassloch (13) in einer länglichen Form auf einem Boden der Kupplungsnut des Behälterkörpers quer zu den Schlitzen des Nadelgehäuses gebildet ist, und ein Luftloch, das ein Austreten von Fluid in dem Behälterkörper verhindert und ein Strömen von Luft ermöglicht, in einem oberen Abschnitt des Kupplungsabschnitts gebildet ist.

2. - Stempelartiger Fluidzufuhrbehälter nach Anspruch 1, wobei eine Nadelsitznut in der Mitte eines Bodens der Dichtungsabdeckung gebildet ist, sodass obere Enden der Mikronadeln darin positioniert sind.

3. - Stempelartiger Flüssigkeitsabgabebehälter nach Anspruch 1, wobei eine aus Gummi gefertigte Bodenkappe an einen Boden des Behälterkörpers gekoppelt ist, um in der Lage zu sein, zum Befüllen von Fluid geöffnet und geschlossen zu werden.

## Revendications

1. - Récipient de distribution de fluide de type tampon, à micro-aiguilles, le récipient de distribution de fluide de type tampon comprenant :
un corps de récipient (10) configuré pour contenir du fluide à l'intérieur de celui-ci, ayant une partie d'accouplement (11) sur un côté extérieur d'une partie supérieure de celui-ci, et ayant une rainure d'accouplement (32) à l'intérieur de la partie supérieure ;
un logement à aiguilles (20) ayant un corps qui est introduit dans la rainure d'accouplement du corps de récipient et qui a des fentes qui sont formées verticalement à travers le corps et disposées parallèlement à gauche et à droite, et dans lesquelles des micro-aiguilles sont introduites, respectivement ;
un couvercle d'étanchéité (30) installé sur un dessus du corps de récipient et empêchant une fuite de fluide sortant des fentes du logement à aiguilles ; et
un bouchon de fermeture (40) accouplé à la partie d'accouplement du corps de récipient,
un trou d'évacuation de fluide (13) étant réalisé en une forme oblongue sur un fond de la rainure d'accouplement du corps de récipient à travers les fentes du logement à aiguilles, et un trou d'air empêchant une fuite de fluide dans le corps de récipient et permettant à de l'air de circuler étant formé dans une partie supérieure de la partie d'accouplement.

2. - Récipient de distribution de fluide de type tampon selon la revendication 1, dans lequel une rainure de siège d'aiguilles est formée à un centre d'un fond du couvercle d'étanchéité, de telle sorte que des extrémités supérieures des micro-aiguilles sont positionnées à l'intérieur de celui-ci.

3. - Récipient de distribution de fluide de type tampon selon la revendication 1, dans lequel un bouchon de fond fait de caoutchouc est accouplé à un fond du corps de récipient de façon à pouvoir être ouvert et fermé pour le remplissage de fluide.
